# EUROPEAN PATENT APPLICATION

(11) **EP 4 414 001 A1**
(43) Date of publication of application: **14.08.2024**
(21) Application number: 22878598.6
(22) Date of filing: 06.10.2022
(51) Int. Cl.: A61K 48/00, A61P 43/00, A61P 17/00, A61Q 19/08, A61K 8/55, A61K 31/7068

(54) **SKIN CARE COMPOSITION AND USE THEREOF, AND SKIN CARE COMPOSITION SOURCE MATERIAL**

(30) Priority: 07.10.2021 JP 2021165433
(71) Applicant: YAMASA CORPORATION, Choshi-shi, Chiba 288-0056 (JP)
(72) Inventor: ISHIGE,Kazuya, Choshi-shi, Chiba 288-0056 (JP)
(74) Representative: Maiwald GmbH
(86) International application number: PCT/JP2022/037513
(87) International publication number: WO 2023/058731

(57) **Abstract**

A skin care composition for suppressing formation of wrinkles caused by photoaging, wherein the composition contains at least one pyrimidine nucleotide or a precursor thereof as an active ingredient.

## Description

### [Field of the Invention]

The present invention relates to a skin care composition and use thereof, as well as a skin care composition raw material. More particularly, the present invention relates to a skin care composition for suppressing the formation of wrinkles caused by photoaging, and use thereof, as well as a skin care composition raw material.

### [Background of the Invention]

In today's aging society, healthy longevity as well as improvement of QOL (Quality of Life) have become issues. Skin beauty in the anti-aging field is very important for improving the quality of life of elderly people, and is receiving increasing attention.

It is known that skin aging that accompanies the aging process includes intrinsic aging (natural aging) and extrinsic aging (Non-Patent Literature 1). Among these, the extrinsic aging caused by sunlight, mainly ultraviolet rays (UV), is called photoaging, and is considered to be the main cause of skin aging.

One of the most important changes in skin appearance that accompany the aging process is the formation of wrinkles. It is believed that 80% of facial wrinkles are caused by photoaging. Furthermore, the formation of wrinkles caused by natural aging and the formation of wrinkles caused by photoaging are qualitatively different; the former is known to form fine and shallow wrinkles, while the latter is known to form deep wrinkles (Non-Patent Literature 2). Therefore, the suppression of the formation of wrinkles caused by photoaging would be important for improving the QOL of elderly people.

The action mechanism that forms the wrinkles caused by photoaging in the skin exposed to UV would be as follows. Epidermal cells exposed to UV secrete excessive amounts of inflammatory cytokines such as interleukin 1α (IL-1α), interleukin 6 (IL-6), and interleukin 8 (IL-8). The inflammatory cytokines excessively secreted from the epidermal cells cause extracellular matrix degrading factors to be secreted from the epidermal cells, fibroblasts, neutrophils, and the like. Local decomposition of the extracellular matrix triggers the formation of wrinkles (Non-Patent Literatures 3 and 4).

Although various raw materials have been investigated for suppressing the formation of wrinkles caused by photoaging, no decisive raw material has yet been discovered. Upon application as a skin care composition, it is considered that a plurality of raw materials with different properties and different action mechanisms are used together. Therefore, there is a need for novel raw materials (e.g., wrinkle formation inhibitors, and the like) that have anti-wrinkle effects.

Nucleotides such as cytidylic acid and their analogs are compounds that are widely contained in living organisms and foods, and can be highly safe materials. The nucleotides and their analogs have been widely considered for application due to their properties such as availability and stability. However, as shown below, there is no useful finding regarding the contribution of cytidylic acid, uridylic acid, and their analogs to suppressing of the formation of wrinkles caused by photoaging.

Patent Literature 1 describes a collagen production promoting composition containing a purine-based nucleic acid-related substance and a pyrimidine-based nucleic acid-related substance. However, the invention substantially disclosed in Patent Literature 1 is the collagen production promoting composition using the combination of adenylic acid and uridylic acid, and there is no substantial disclosure regarding the use of uridylic acid alone and the use of cytidylic acid. In addition, collagen production and photoaging induced by inflammatory cytokines are completely different pathways. That is, Patent Literature 1 is in a different technical field from the present invention which relates to the suppression of the formation of wrinkles caused by photoaging, and Patent Literature 1 does not disclose the suppressing of the formation of wrinkles caused by photoaging.

Patent Literature 2 describes a method for treating or preventing tissue damage caused by systemic inflammatory response syndrome caused by infection with bacteria, viruses, or the like, by administering a pyrimidine nucleotide precursor. However, substantially disclosed is the use of uridine or triacetyluridine, and there is no substantial disclosure regarding the application of cytidine, cytidylic acid, or uridylic acid. Moreover, Patent Literature 2 discloses a systemic anti-inflammatory agent, and it does not disclose photoaging in the skin. Furthermore, it discloses applications as oral preparations and injections, while it does not disclose or suggest any application as external preparations. In addition, exogenous substances derived from infected microorganisms are inflammation-inducing substances called PAMPs (Pathogen-Associated Molecular Pattern Molecules), but substances that would be related to photoaging are inflammation-inducing substances called DAMPs (Damage-Associated Molecular Pattern Molecules). That is, Patent Literature 2 is in a different technical field from the present invention which relates to the suppression of the formation of wrinkles caused by photoaging.

Patent Literature 3 describes a composition for preventing skin pigmentation by cytidine and an analog thereof. There is no substantial disclosure regarding uridine, uridylic acid, and cytidylic acid. It discloses the action mechanism for preventing pigmentation by inhibiting the activity of sialyltransferase to inhibit the synthesis of membrane-bound complex carbohydrates found in the dendrites of melanocytes, and suppressing the migration from melanocytes involved by the carbohydrates to keratinocytes. The action mechanism described in Patent Literature 3 is a completely different pathway from the formation of wrinkles caused by photoaging. That is, Patent Literature 3 is in a different technical field from the present invention which relates to the suppression of the formation of wrinkles caused by photoaging.

Patent Literature 4 describes effects of promoting hyaluronic acid production and of preventing/treating atopic dermatitis by a uridine phosphate ester or a physiologically acceptable salt thereof. Patent Literature 4 does not substantially disclose the use of cytidylic acid. Furthermore, Patent Literature 4 merely demonstrates the promotion of hyaluronic acid production, and there is no example to support the fact that atopic dermatitis can be prevented/treated when it is used as a skin care agent. In addition, as described in Non-Patent Literature 5, the atopic dermatitis is an immune response caused by invading antigens or stimulative substances into the skin, and activating innate immunity and acquired immunity, resulting in induction of Th2-dominant immune response, and its action mechanism is completely different from that of photoaging. As for the application as the skin care agent, the prevention/treatment of atopic dermatitis is a medical field or a technical field similar to medicine, and is completely different from the present invention relating to the suppression of photoaging, which is appealed in cosmetics and the like.

As described above, the inventions disclosed in Patent Literatures 1 to 4 and other known documents have no disclosure or suggestion of the phenomenon targeted by the present invention (the suppression of the formation of wrinkles caused by photoaging) and its action mechanism, and are in the different technical fields.

### [Citation List]

### [Patent Literatures]

[PTL 1] Japanese Patent Application Publication No. 2010-215648 A
[PTL 2] Japanese Patent Application Publication No. H08-503699 A
[PTL 3] Japanese Patent Application Publication No. 2016-510760 A
[PTL 4] WO 2019/021988 A1

### [Non-Patent Literatures]

[NON-PTL 1] Journal of Japanese Cosmetic Science Society, Vol. 41, No. 3, pp. 240-243 (2017)
[NON-PTL 2] Journal of Japanese Cosmetic Science Society, Vol. 41, No. 3, pp. 244-245 (2017)
[NON-PTL 3] Journal of Japanese Cosmetic Science Society, Vol. 37, No. 1, pp. 11-16 (2013)
[NON-PTL 4] Journal of Japanese Cosmetic Science Society, Vol. 43, No. 2, pp. 109-112 (2019)
[NON-PTL 5] Allergy, vol. 68, No. 7, p.815-822 (2019)

### [Summary of the Invention]

### [Problem to be Solved by the Invention]

In an aspect of the present invention, an object of the present invention is to provide a novel and highly safe skin care composition and a method for using the same, which have an effect of suppressing the formation of wrinkles caused by photoaging. In another aspect, an object of the present invention is to provide a skin care composition raw material that can be used as a raw material for the skin care composition.

### [Means for Solving the Problem]

As a result of intensive studies to achieve the above objects, the present inventors have been newly found that a pyrimidine nucleotide or a precursor thereof has properties useful for suppressing the formation of wrinkles caused by photoaging, and they have achieved the present invention.

### [Effects of the Invention]

In an aspect, according to the present invention, it is possible to provide a novel and highly safe skin care composition and a method for using the same, which have an effect of suppressing the formation of wrinkles caused by photoaging. Also, according to the present invention, in another aspect, it is possible to provide a skin care composition raw material that can be used as a raw material for the skin care composition.

### [Brief Description of the Drawings]

[Fig. 1]

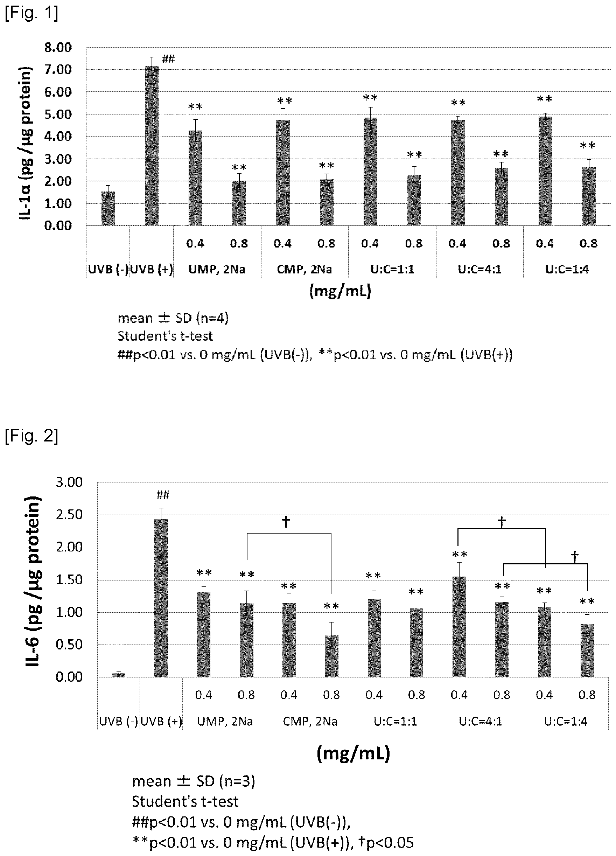

   Fig. 1 shows an amount of IL-1α (pg/pg-protein) in a culture supernatant of human epidermal keratinocytes (keratinocytes) obtained in Example 1. In the figure, the UVB (-) means that no UV treatment was performed and no sample treatment was performed as a control. The UVB (+) means that a UV treatment was performed as a control, but no sample treatment was performed. The bar labeled as UMP, 2Na 0.4 means that it was treated with 0.4 mg/mL of UMP,2Na in addition to the UV treatment. Similarly, the bar labeled as UMP,2Na 0.8 means that it was treated with 0.8 mg/mL of UMP,2Na, and the bar labeled as CMP,2Na 0.4 means that it was treated with 0.4 mg/mL of CMP,2Na, and the bar labeled as U:C=1:1 0.4 means that it was treated with 0.4mg/mL of a 1:1 mass ratio mixture of UMP,2Na and CMP,2Na. Other samples are also described in the same format. The symbol ## means that the p value is less than 0.01 when compared with UVB (-) by Student's t-test. The symbol ** means that the p-value is less than 0.01 when compared with UVB (+) by Student's t-test.
[Fig. 2]
   Fig. 2 shows an amount of IL-6 (pg/µg-protein) in a culture supernatant of human epidermal keratinocytes (keratinocytes) obtained in Example 1. In the figure, the UVB (-) means that no UV treatment was performed and no sample treatment was performed as a control. The UVB (+) means that a UV treatment was performed as a control, but no sample treatment was performed. The bar labeled as UMP, 2Na 0.4 means that it was treated with 0.4 mg/mL of UMP,2Na in addition to the UV treatment. Similarly, the bar labeled as UMP,2Na 0.8 means that it was treated with 0.8 mg/mL of UMP,2Na, and the bar labeled as CMP,2Na 0.4 means that it was treated with 0.4 mg/mL of CMP,2Na, and the bar labeled as U:C=1:1 0.4 means that it was treated with 0.4mg/mL of a 1:1 mass ratio mixture of UMP,2Na and CMP,2Na. Other samples are also described in the same format. The symbol ## means that the p value is less than 0.01 when compared with UVB (-) by Student's t-test. The symbol ** means that the p-value is less than 0.01 when compared with UVB (+) by Student's t-test. The symbol † means that the p-value is less than 0.05 when comparing the results between two samples using Student's t-test.
[Fig. 3]

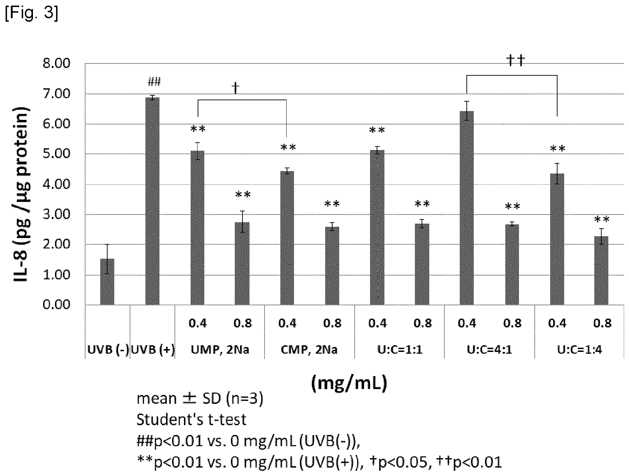

   Fig. 3 shows an amount of IL-8 (pg/µg-protein) in a culture supernatant of human epidermal keratinocytes (keratinocytes) obtained in Example 1. In the figure, the UVB (-) means that no UV treatment was performed and no sample treatment was performed as a control. The UVB (+) means that a UV treatment was performed as a control, but no sample treatment was performed. The bar labeled as UMP, 2Na 0.4 means that it was treated with 0.4 mg/mL of UMP,2Na in addition to the UV treatment. Similarly, the bar labeled as UMP,2Na 0.8 means that it was treated with 0.8 mg/mL of UMP,2Na, and the bar labeled as CMP,2Na 0.4 means that it was treated with 0.4 mg/mL of CMP,2Na, and the bar labeled as U:C=1:1 0.4 means that it was treated with 0.4mg/mL of a 1:1 mass ratio mixture of UMP,2Na and CMP,2Na. Other samples are also described in the same format. The symbol ## means that the p value is less than 0.01 when compared with UVB (-) by Student's t-test. The symbol ** means that the p-value is less than 0.01 when compared with UVB (+) by Student's t-test. The symbol † means that the p-value is less than 0.05 when comparing the results between two samples using Student's t-test. The symbol †† means that the p value is less than 0.01 when comparing the results between two samples using Student's t test.
[Fig. 4]

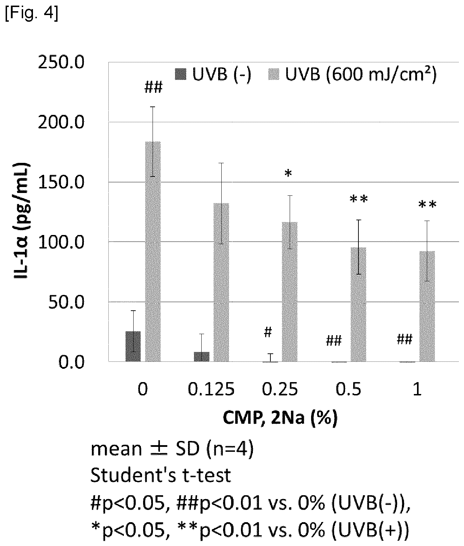

   Fig. 4 shows an amount of IL-1α (pg/mL) in a culture supernatant of a three-dimensional cultured human epidermis obtained in Example 2. In the figure, the dark-colored bars mean those which were not subjected to the UV treatment, and the light-colored bars mean those which were subjected to the UV treatment (600 mJ/cm²). The bar labeled as 0 means that the UV treatment was performed as a control, but no CMP treatment was performed. The bar labeled as 0.125 means that it was treated with 0.125 (w/v%) of CMP,2Na in addition to the UV treatment. Similarly, the bar labeled as 0.25 means that it was treated with 0.25 (w/v%) of CMP,2Na, and the bar labeled as 0.5 means that it was treated with 0.5 (w/v%) of CMP,2Na, and the bar labeled as 1 means that it was treated with 1.0 (w/v%) of CMP,2Na. The symbol # means that the p-value is less than 0.05 when compared with CMP,2Na 0 (w/v%) UVB (-) using Student's t-test, and the symbol ## means the p-value is less than 0.01. The * symbol means that the p value is less than 0.05 when compared with CMP,2Na 0 (w/v%) UVB (600 mJ/cm²) using Student's t test, and the ** symbol means that the p value is less than 0.01.
[Fig. 5]

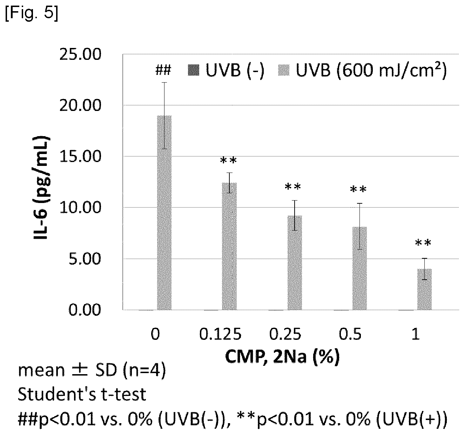

   Fig. 5 shows an amount of IL-6 (pg/mL) in a culture supernatant of a three-dimensional cultured human epidermis obtained in Example 2. In the figure, the dark-colored bars mean those which were not subjected to the UV treatment, and the light-colored bars mean those which were subjected to the UV treatment (600 mJ/cm²). The bar labeled as 0 means that the UV treatment was performed as a control, but no CMP treatment was performed. The bar labeled as 0.125 means that it was treated with 0.125 (w/v%) of CMP,2Na in addition to the UV treatment. Similarly, the bar labeled as 0.25 means that it was treated with 0.25 (w/v%) of CMP,2Na, and the bar labeled as 0.5 means that it was treated with 0.5 (w/v%) of CMP,2Na, and the bar labeled as 1 means that it was treated with 1.0 (w/v%) of CMP,2Na. The symbol ## means that the p-value is less than 0.01 when compared with CMP, 2Na 0 (w/v%) UVB (-) using Student's t-test. The ** symbol means that the p value is less than 0.01 when compared with CMP, 2Na 0 (w/v%) UVB (600 mJ/cm²) using Student's t test.
[Fig. 6]

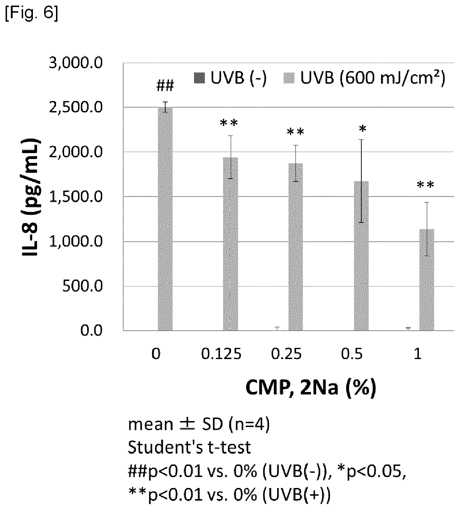

   Fig. 6 shows an amount of IL-8 (pg/mL) in a culture supernatant of a three-dimensional cultured human epidermis obtained in Example 2. In the figure, the dark-colored bars mean those which were not subjected to the UV treatment, and the light-colored bars mean those which were subjected to the UV treatment (600 mJ/cm²). The bar labeled as 0 means that the UV treatment was performed as a control, but no CMP treatment was performed. The bar labeled as 0.125 means that it was treated with 0.125 (w/v%) of CMP,2Na in addition to the UV treatment. Similarly, the bar labeled as 0.25 means that it was treated with 0.25 (w/v%) of CMP,2Na, and the bar labeled as 0.5 means that it was treated with 0.5 (w/v%) of CMP,2Na, and the bar labeled as 1 means that it was treated with 1.0 (w/v%) of CMP,2Na. The symbol ## means that the p-value is less than 0.01 when compared with CMP, 2Na 0 (w/v%) UVB (-) using Student's t-test. The * symbol means that the p value is less than 0.05 when compared with CMP, 2Na 0 (w/v%) UVB (600 mJ/cm²) using Student's t test, and the ** symbol means that the p value is less than 0.01.

### [Detailed Description of the Invention]

Hereinafter, embodiments of the present invention will be specifically described. It is to understand that the present invention is not limited to the following embodiments, and those which appropriately added changes, improvements and the like to the following embodiments based on knowledge of a person skilled in the art without departing from the spirit of the present invention fall within the scope of the present invention.

The present invention relates to a skin care composition containing a pyrimidine nucleotide or a precursor thereof as an active ingredient.

As used herein, the term "skin care composition" refers to a product (e.g., a cosmetic, pharmaceutical, quasi-drug) that is applied (e.g., painted, sprayed, pasted, or the like) to the skin. Specifically, the skin care composition can be used as a cosmetic, a skin care pharmaceutical, a skin care quasi-drug, and the like. Among these, the skin care composition is preferably a cosmetic from the viewpoint of suppressing the formation of wrinkles caused by photoaging of the skin on a daily basis. The cosmetic includes general skin cosmetics, as well as various hair cosmetics such as hair growth tonics, hair restoration tonics, or shampoos, conditioners, and hair lotions (including tonics and liquids) that are effective for hair growth or hair restoration.

It should be noted that the skin care composition may be referred to as a cosmetic composition, a skin external agent, and the like.

As used herein, the pyrimidine nucleotide means cytidylic acid and/or uridylic acid.

The cytidylic acid (cytidine monophosphate, cytidine 5'-phosphate, CMP) is a compound represented by CAS Registry Number 63-37-6. When cytidylic acid is mentioned herein, salts of cytidylic acid are also included.

When a mass of cytidylic acid is described herein, it represents a mass when converted to disodium cytidylate (CMP,2Na). When a concentration (%) of a cytidylic acid solution is mentioned herein, it is a mass volume percent concentration (w/v%) unless otherwise specified, and a mass converted to CMP,2Na is used as the mass of cytidylic acid. If a salt other than the disodium salt is selected, and if it is a free acid that does not form a salt, it is a mass when converted to CMP,2Na, based on the amount of substance of the cytidylic acid.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to disodium uridylate (UMP,2Na). When a concentration (%) of a uridylic acid solution is mentioned herein, it is a mass volume percent concentration (w/v%) unless otherwise specified, and a mass converted to UMP,2Na is used as the mass of uridylic acid. If a salt other than the disodium salt is selected, and if it is a free acid that does not form a salt, it is a mass when converted to UMP,2Na, based on the amount of substance of the uridylic acid.

As used herein, a pyrimidine nucleotide precursor means a compound that can be metabolized to the pyrimidine nucleotide, i.e., cytidylic acid and/or uridylic acid. Whether a compound is included in the pyrimidine nucleotide precursor is determined by the presence or absence of knowledge that the compound is converted to the pyrimidine nucleotide. Specifically, cytidine diphosphate, cytidine triphosphate, uridine diphosphate, and uridine triphosphate, which are known to be degraded to cytidylic acid and/or uridylic acid by the action of ectonucleotidases and like (Clinical Chemistry 33: 11-18, 2004), and cytidine, cytosine, uridine, and uracil, which are known to be phosphorylated to cytidylic acid and/or uridylic acid by the action of kinases (J Biol Chem. 1969 Apr 25; 244(8): 2204-9.) are exemplified as the pyrimidine nucleotide precursors as used herein.

As used herein, the "photoaging" refers to general skin aging phenomena caused by exposure of epidermal cells to UV or sunlight. Specifically, it includes secretion of inflammatory cytokines such as interleukin 1α (IL-1α), interleukin 6 (IL-6), and interleukin 8 (IL-8), secretion of extracellular matrix degrading factors from epidermal cells and fibroblasts, induced by secretion of the inflammatory cytokines, and changes of appearances caused by these factors, such as wrinkles, macula, and sagging.

As used herein, the "formation of wrinkles caused by photoaging" refers to a series of processes: exposure of epidermal cells to UV or sunlight, secretion of inflammatory cytokines such as interleukin 1α (IL-1α), interleukin 6 (IL-6), and interleukin 8 (IL-8), secretion of extracellular matrix degrading factors from epidermal cells and fibroblasts induced by the secretion of the inflammatory cytokines, and the formation of wrinkles caused thereby. As described in Non-Patent Literatures 1 and 2, the mechanism of occurrence of "the formation of wrinkles caused by photoaging" is different from that of normal natural aging, and the severity and frequency of the formation of wrinkles caused are known to be significantly higher.

As used herein, the term "inflammatory cytokine" refers to a low-molecular protein secreted from cells that shows a physiological effect of inducing inflammation. Specific examples of inflammatory cytokine include IL-1α, IL-1β, IL-6, IL-8, IL-11, IL-17, IL-18, IFN-α, IFN-β, IFN-γ, TNF-α and TNF-β.

As used herein, the anti-inflammatory effect of the present invention is evaluated by quantifying amounts of inflammatory cytokines produced. As the inflammatory cytokines used for evaluation, IL-1α, IL-6, and IL-8 are selected because of the accumulated knowledge regarding the formation of wrinkles caused by photoaging. A specific quantitative method will be described in the Example section below.

Examples of the pyrimidine nucleotides or precursors thereof in the present invention include, as described above, cytidine, cytidylic acid, cytidine diphosphate, cytidine triphosphate, uridine, uridylic acid, uridyl diphosphate, and uridyl triphosphate. Among them, cytidylic acid or a precursor thereof is preferable, cytidylic acid is more preferable, in terms of exhibiting a higher IL-6 and IL-8 secretion suppressing effect.

The concept of cytidylic acid as used herein includes salts as described above. The salts of cytidylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Preferable salts of cytidylic acid may be alkali metal salts such as sodium salts. Specific examples of such alkali metal salts include monosodium cytidylate and disodium cytidylate. Among them, disodium cytidylate is preferred.

The concept of uridylic acid as used herein includes salts as described above. The salts of uridylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Preferable salts of uridylic acid may be alkali metal salts such as sodium salts. Specific examples of such alkali metal salts include monosodium uridylate and disodium uridylate. Among them, disodium uridylate is preferred.

The content of the pyrimidine nucleotide or the precursor thereof in the skin care composition according to the present invention is preferably 0.125% or more in terms of exerting the effect of suppressing IL-6 and IL-8 secretion, and more preferably 0.25% or more in terms of also exerting the effect of suppressing IL-1α secretion. Further, from the viewpoint of ease of handling, the content of the pyrimidine nucleotide or the precursor thereof is preferably 10% or less.

In the skin care composition according to the present invention, the pyrimidine nucleotide or the precursor thereof as the active ingredient may be used alone, or two or more types of pyrimidine nucleotides or precursors thereof may be simultaneously used, or the pyrimidine nucleotide or the precursor thereof may be used in combination with other active ingredients. Examples of the active ingredients other than pyrimidine nucleotide precursor include, but not limited to, purine nucleotides such as adenylic acid.

There is no particular limitation on the origins of the pyrimidine nucleotides or precursors thereof, and those derived from natural products such as yeast, bacteria, fish and shellfish, animals, and plants are suitable.

The skin care composition according to the present invention may be prepared in various forms by combining pharmaceutically or cosmetically acceptable bases and carriers in addition to the above ingredients. Conventionally known bases and carriers can be used as the pharmaceutically or cosmetically acceptable bases and carriers. Also, the skin care composition according to the present invention may optionally contain various known ingredients that will be formulated in external compositions applied to the skin or mucous membranes, such as cosmetics or external pharmaceuticals and quasi-drugs. Such ingredients include, for example, surfactants, coloring matters (dyes, pigments), fragrances, preservatives, bactericides (antibacterial agents), thickening agents, antioxidants, sequestering agents, cooling agents, deodorants, moisturizers, UV absorbers, UV scattering agents, vitamins, plant extracts, skin astringents, anti-inflammatory agents (antiphlogistic agents), whitening agents, cell activators, vasodilator agents, blood circulation promoters, skin function enhancers, and the like.

Among the above ingredients, specific examples of the surfactants include anionic surfactants such as higher fatty acid soaps, alkyl sulfate ester salts, polyoxyethylene alkyl ether sulfates, alkyl ether phosphate ester salts, N-acylamino acid salts, and acyl N-methyl taurine salts; cationic surfactants such as alkyltrimethylammonium chloride, and dialkyldimethylammonium chloride; amphoteric surfactants such as alkyl dimethyl amino acetic acid betaine, alkyl amido dimethyl amino acetic acid betaine, and 2-alkyl-N-carboxy-N-hydroxyimidazolinium betaine; nonionic surfactants such as polyoxyethylene type, polyhydric alcohol ester type, and ethylene oxide-propylene oxide block copolymers. Further, surfactants belonging to polymer surfactants or natural surfactants can also be used without limitation.

Specific examples of preservatives include ethyl paraoxybenzoate, salicylic acid, and sorbic acid. Specific examples of the thickening agent include xanthan gum, sodium carboxymethylcellulose, and carboxyvinyl polymers. Specific examples of the sequestering agent include sodium salt of ethylenediaminetetraacetic acid, phosphoric acid, and citric acid.

Specific examples of the moisturizers include polyethylene glycol, propylene glycol, glycerin, 1,3-butylene glycol, xylitol, sorbitol, maltitol, chondroitin sulfate, hyaluronic acid, mucoitinsulfuric acid, charonic acid, atelocollagen, sodium lactate, bile salts, dl-pyrrolidone carboxylate, short-chain soluble collagen, diglycerin (EO) PO adducts, chestnut rose extracts, yarrow extracts, and melilot extracts.

Specific examples of the vitamins include classes of vitamin A such as vitamin A oil, retinol, retinol acetate, and retinol palmitate, classes of vitamin B2 such as riboflavin, riboflavin butyrate, and flavin adenine nucleotides, classes of vitamin B6 such as pyridoxine hydrochloride, and pyridoxine dioctanoate, classes of vitamin C such as L-ascorbic acid, L-ascorbyl dipalmitate, L-ascorbic acid-2-sodium sulfate, and dl-α-tocopherol-L-ascorbic acid phosphate diester dipotassium, pantothenic acids such as calcium pantothenate, D-pantothenyl alcohol, pantothenyl ethyl ether, and acetyl pantothenyl ethyl ether, classes of vitamin D such as ergocalciferol and cholecalciferol, nicotinic acids such as nicotinic acid, benzyl nicotinate, and nicotinamide, classes of vitamin E such as dl-α-tocopherol, dl-α-tocopherol acetate, dl-α-tocopherol nicotinate, and dl-α-tocopherol succinate, and vitamin P, biotin, and the like.

The form of the skin care composition according to the present invention is not particularly limited as long as it can be applied to the skin or mucous membranes, and examples include pastes, mousses, gels, liquids, emulsions, suspensions, creams, ointments, sheets, aerosols, sprays, and liniments. In particular, when it is used as a cosmetic, examples include lotions; emulsions such as emollient emulsions, milky lotions, nourishing emulsions, and cleansing emulsions; creams such as emollient creams, massage creams, cleansing creams, and makeup creams. Further, in particular, when it is used as a hair care product such as hair growth agents and hair restoration agents, examples include tonics, hair creams, hair lotions, aerosols (sprays), mousses, shampoos, conditioners, and liquids.

The skin care composition according to the present invention can be used by directly applying, spraying, or pasting onto the skin or mucous membranes. The rate of use can be appropriately selected depending on the user's (human) age, sex, purpose, severity of symptoms in the affected area, and the like, and is not particularly limited. For example, an effective amount to suppress the formation of wrinkles caused by photoaging may be transdermally administered to the skin from once per day to five times per day.

An amount of application when applying the skin care composition according to the present invention is preferably a single dose of 1 mg or more as converted to cytidylic acid or uridylic acid, more preferably a single dose of 1 to 100 mg, more preferably 5 to 80 mg, and even more preferably 10 to 50 mg.

As shown in the Example section as described below, the skin care composition according to the present invention suppresses excessive secretion of inflammatory cytokines from epidermal cells exposed to UV. By suppressing excessive secretion of inflammatory cytokines, inflammation of the skin can be directly suppressed. Furthermore, the skin care composition according to the present invention can suppress downstream phenomena caused by excessive secretion of inflammatory cytokines, such as secretion of extracellular matrix degrading factors from epidermal cells, fibroblasts, neutrophils, and the like, and/or the formation of wrinkles.

As a secondary effect of suppressing the formation of wrinkles caused by photoaging, the present invention can also prevent other photoaging phenomena. The secondary effects that can be exerted by the present invention include moisturizing, improvement of skin texture, anti-sagging, anti-dullness, and anti-spot.

As is clear from the above contents, the present invention is based on the findings that the pyrimidine nucleotide or the precursor thereof has a novel property of suppressing the excessive secretion of inflammatory cytokines from UV-exposed epidermal cells, and that the property allows the pyrimidine nucleotide to be suitable for use in the skin care composition (particularly the skin care compositions having anti-inflammatory effects and the effect of suppressing the formation of wrinkles caused by photoaging).

Therefore, the pyrimidine nucleotide or the precursor thereof can also be used as a raw material for the skin care composition (hereinafter referred to as a "skin care composition raw material"). That is, the skin care composition raw material can be used as at least one agent selected from wrinkle formation inhibitors for inhibiting the formation of wrinkles caused by photoaging, inflammatory cytokine production inhibitors for inhibiting inflammatory cytokine production caused by ultraviolet rays, and anti-inflammatory agents for inhibiting inflammation caused by ultraviolet rays.

It should be noted that the skin care composition raw material may contain components other than the pyrimidine nucleotide or its precursor (for example, a solvent used during the synthesis of the pyrimidine nucleotide or its precursor) to the extent that the function of the pyrimidine nucleotide or its precursor is not inhibited. Furthermore, the skin care composition raw material is not particularly limited, and may be either in the form of a solid or a liquid.

### [Examples]

While the present invention will be described below with reference to Examples, the present invention is not limited to these Examples in any way.

### (Example 1) Anti-inflammatory Evaluation Using Normal Human Keratinocytes

The effects of using various nucleotides on photoaging of the skin were evaluated using normal human keratinocytes. The effects of various nucleotides on inflammatory cytokines induced by UV irradiation of normal human keratinocytes were evaluated. Known inflammatory cytokines IL-1α, IL-6, and IL-8 were used as the inflammatory cytokines to be evaluated.

The experiment was conducted according to the following procedure.

### (UV Irradiation of Normal Human Keratinocytes and Treatment with Various Nucleotides)

Normal human keratinocytes (NHEK; manufactured by Kurabo Industries, Ltd.) were seeded in a 96-well plate at a density of 2.5 × 10⁴ cells/well and cultured at 37°C for 24 hours. The medium was replaced with 100 µL of sample-containing medium (HuMedia-KG2; manufactured by Kurabo Industries, Ltd.) and cultured for 24 hours. After removing the sample-containing medium, the plate was washed with 100 µL of Hanks balanced salt solution (HBSS (-)) and irradiated with 20 mJ/cm² using a UVB lamp (TL20W/12RS; manufactured by PHILIPS). After washing with HBSS (-), the medium was replaced with 100 µL of fresh medium and cultured for 24 hours. After culturing, the culture supernatant was collected. The sample solutions used were 0.8 (mg/mL) of UMP,2Na 0.4, 0.8 (mg/mL)of CMP,2Na 0.4, 0.8 (mg/mL) of U:C=1:1 (mass ratio 1:1 mixture of UMP,2Na and CMP,2Na) 0.4, 0.8 (mg/mL) of U:C=4:1 (mass ratio 4:1 mixture of UMP,2Na and CMP,2Na) 0.4, and 0.8 (mg/mL) of U:C=1:4 (mass ratio 1:4 mixture of UMP,2Na and CMP,2Na) 0.4.

### (Quantification of Inflammatory Cytokines)

The culture supernatant of human keratinocytes was collected, and amounts of IL-1α, IL-6, and IL-8 were quantified by ELISA.

The quantitative results of IL-1α, IL-6, and IL-8 are shown in Figs. 1, 2, and 3, respectively.

The quantitative results of IL-1α (Fig. 1) revealed that the addition of uridylic acid, cytidylic acid suppressed the amount of IL-1α produced. It was revealed that both uridylic acid and cytidylic acid increased the inhibitory effect in a concentration-dependent manner. No significant difference was observed when comparing uridylic acid with cytidylic acid.

The quantitative results of IL-6 (Fig. 2) revealed that the addition of uridylic acid, cytidylic acid suppressed the amount of IL-6 produced. It was revealed that both uridylic acid and cytidylic acid increased the inhibitory effect in a concentration-dependent manner. A significant difference was observed when comparing uridylic acid with cytidylic acid. Furthermore, when comparing mixtures of uridylic acid and cytidylic acid, as the cytidylic acid concentration increased, the inhibitory effect on IL-6 production became stronger. These results revealed that cytidylic acid had a particularly remarkable effect of inhibiting IL-6 production.

The quantitative results of IL-8 (Fig. 3) revealed that the addition of uridylic acid, cytidylic acid suppressed the amount of IL-8 produced. It was revealed that both uridylic acid and cytidylic acid increased the inhibitory effect in a concentration-dependent manner. A significant difference was observed when comparing uridylic acid with cytidylic acid. Furthermore, when comparing mixtures of uridylic acid and cytidylic acid, as the concentration of cytidylic acid increased, the inhibitory effect of IL-8 production became stronger. These results revealed that cytidylic acid had a particularly remarkable effect of inhibiting IL-8 production.

The results of this Example revealed that uridylic acid and cytidylic acid had the effect of suppressing the production of inflammatory cytokines. Among these, it was revealed that cytidylic acid had a remarkable effect of suppressing the production of inflammatory cytokines.

In addition, the anti-inflammatory evaluation of the purine nucleotides, adenylic acid, guanylic acid, and inosinic acid, was performed using a similar experimental system, but only a lower effect of inhibiting inflammatory cytokine production than cytidylic acid and uridylic acid was observed.

### (Example 2) Anti-inflammatory Evaluation Using Three-Dimensional Cultured Human Epidermis

To reproduce the phenomenon that occurs in actual epidermis, three-dimensional cultured human epidermis was used to evaluate the suppression effect of cytidylic acid on inflammatory cytokines induced by UV irradiation. In addition, to verify the relationship between the amount of cytidylic acid and the suppression effect on inflammatory cytokine production, the effects of varying the amount of cytidylic acid were evaluated.

### (Acquisition of Culture Supernatant of Three-dimensional Cultured Human Epidermis)

Three-dimensional cultured human epidermis (LabCyte EPI MODEL 24; manufactured by J TEC) was set in a 24-well plate to which 500 µL of the attached assay medium was added, and acclimated overnight at 37°C. 50 µL of sample-containing PBS (-) was applied from the stratum corneum side and cultured for 24 hours. After removing the sample-containing PBS (-), the plate was washed with PBS (-) and irradiated with 600 mJ/cm² using a UVB lamp (TL20W/12RS; manufactured by PHILIPS). The medium was replaced with a fresh medium, 50 µL of sample-containing PBS (-) was newly applied from the stratum corneum side, and further cultured for 48 hours. After culturing, the culture supernatant was collected. The concentrations of cytidylic acid in the respective samples were 0, 0.125, 0.25, 0.5, and 1.0 (w/v%), respectively. Each inflammatory cytokine contained in the supernatant was quantified by ELISA.

The quantitative results of IL-1α, IL-6, and IL-8 are shown in Figs. 4, 5, and 6, respectively.

For IL-1α, a significant IL-1α production inhibitory effect was observed in the treatments with 0.25% or more of cytidylic acid.

For IL-6, a significant IL-6 production inhibitory effect was observed in treatments with 0.125% or more of cytidylic acid.

For IL-8, a significant IL-8 production inhibitory effect was observed in treatments with 0.125% or more of cytidylic acid.

In other words, a suppression effect on inflammatory cytokine production was observed under conditions of 0.125% or more of cytidylic acid, and in particular, significant inhibitory effects of all IL-1α, IL-6, and IL-8 were observed under conditions of 0.25% or more of cytidylic acid.

Based on the above results, the present invention can have the following aspects:
[1] A skin care composition for suppressing formation of wrinkles caused by photoaging, wherein the composition comprises at least one pyrimidine nucleotide or a precursor thereof as an active ingredient.
[2] A skin care composition for suppressing inflammatory cytokine production caused by ultraviolet rays, wherein the composition comprises at least one pyrimidine nucleotide or a precursor thereof as an active ingredient.
[3] A skin care composition for suppressing inflammation caused by ultraviolet rays, wherein the composition comprises at least one pyrimidine nucleotide or a precursor thereof as an active ingredient.
[4] The skin care composition according to [1], for suppressing inflammatory cytokine production caused by ultraviolet rays.
[5] The skin care composition according to [1] or [4], for suppressing inflammation caused by ultraviolet rays.
[6] The skin care composition according to any one of [1] to [5], wherein the composition has a concentration of the pyrimidine nucleotide or the precursor thereof of 0.125 to 10.0 w/v%.
[7] The skin care composition according to any one of [1] to [6], wherein the pyrimidine nucleotide is cytidylic acid.
[8] A method for suppressing formation of wrinkles caused by photoaging, using a skin care composition comprising at least one pyrimidine nucleotide or a precursor thereof as an active ingredient.
[9] A method for suppressing inflammatory cytokine production caused by ultraviolet rays, using a skin care composition comprising at least one pyrimidine nucleotide or a precursor thereof as an active ingredient.
[10] A method for suppressing inflammation caused by ultraviolet rays, using a skin care composition comprising at least one pyrimidine nucleotide or a precursor thereof as an active ingredient.
[11] The method according to any one of [8] to [10], wherein the pyrimidine nucleotide or the precursor thereof is administered in a single dose of 1 to 100 mg.
[12] The method according to any one of [8] to [11], wherein a concentration of the pyrimidine nucleotide or the precursor thereof in the skin care composition is 0.125 to 10.0 w/v%.
[13] Use of a skin care composition comprising at least one pyrimidine nucleotide or a precursor thereof as an active ingredient for suppressing formation of wrinkles caused by photoaging.
[14] Use of a skin care composition comprising at least one pyrimidine nucleotide or a precursor thereof as an active ingredient for suppressing inflammatory cytokine production caused by ultraviolet rays.
[15] Use of a skin care composition comprising at least one pyrimidine nucleotide or a precursor thereof as an active ingredient for suppressing inflammation caused by ultraviolet rays.
[16] The use according to any one of [13] to [15], wherein a single dose of the pyrimidine nucleotide or the precursor thereof administered is 1 to 100 mg.
[17] The use according to any one of [13] to [16], wherein a concentration of the pyrimidine nucleotide or the precursor thereof in the skin care composition is 0.125 to 10.0 w/v%.
[18] A skin composition raw material comprising at least one pyrimidine nucleotide or a precursor thereof.
[19] The skin care composition raw material according to [18], wherein the skin care composition raw material is used as at least one agent selected from a wrinkle formation inhibitor for suppressing formation of wrinkles caused by photoaging, an inflammatory cytokine production inhibitor for suppressing inflammatory cytokine production caused by ultraviolet rays, and an inflammation inhibitor for suppressing inflammation caused by ultraviolet rays.

## Claims

1. A skin care composition for suppressing formation of wrinkles caused by photoaging, wherein the composition comprises at least one pyrimidine nucleotide or a precursor thereof as an active ingredient.

2. A skin care composition for suppressing inflammatory cytokine production caused by ultraviolet rays, wherein the composition comprises at least one pyrimidine nucleotide or a precursor thereof as an active ingredient.

3. A skin care composition for suppressing inflammation caused by ultraviolet rays, wherein the composition comprises at least one pyrimidine nucleotide or a precursor thereof as an active ingredient.

4. The skin care composition according to claim 1, for suppressing inflammatory cytokine production caused by ultraviolet rays.

5. The skin care composition according to claim 1 or 4, for suppressing inflammation caused by ultraviolet rays.

6. The skin care composition according to any one of claims 1 to 5, wherein the composition has a concentration of the pyrimidine nucleotide or the precursor thereof of 0.125 to 10.0 w/v%.

7. The skin care composition according to any one of claims 1 to 6, wherein the pyrimidine nucleotide is cytidylic acid.

8. A method for suppressing formation of wrinkles caused by photoaging, using a skin care composition comprising at least one pyrimidine nucleotide or a precursor thereof as an active ingredient.

9. A method for suppressing inflammatory cytokine production caused by ultraviolet rays, using a skin care composition comprising at least one pyrimidine nucleotide or a precursor thereof as an active ingredient.

10. A method for suppressing inflammation caused by ultraviolet rays, using a skin care composition comprising at least one pyrimidine nucleotide or a precursor thereof as an active ingredient.

11. The method according to any one of claims 8 to 10, wherein the pyrimidine nucleotide or the precursor thereof is administered in a single dose of 1 to 100 mg.

12. The method according to any one of claims 8 to 11, wherein a concentration of the pyrimidine nucleotide or the precursor thereof in the skin care composition is 0.125 to 10.0 w/v%.

13. Use of a skin care composition comprising at least one pyrimidine nucleotide or a precursor thereof as an active ingredient for suppressing formation of wrinkles caused by photoaging.

14. Use of a skin care composition comprising at least one pyrimidine nucleotide or a precursor thereof as an active ingredient for suppressing inflammatory cytokine production caused by ultraviolet rays.

15. Use of a skin care composition comprising at least one pyrimidine nucleotide or a precursor thereof as an active ingredient for suppressing inflammation caused by ultraviolet rays.

16. The use according to any one of claims 13 to 15, wherein a single dose of the pyrimidine nucleotide or the precursor thereof administered is 1 to 100 mg.

17. The use according to any one of claims 13 to 16, wherein a concentration of the pyrimidine nucleotide or the precursor thereof in the skin care composition is 0.125 to 10.0 w/v%.

18. A skin composition raw material comprising at least one pyrimidine nucleotide or a precursor thereof.

19. The skin care composition raw material according to claim 18, wherein the skin care composition raw material is used as at least one agent selected from a wrinkle formation inhibitor for suppressing formation of wrinkles caused by photoaging, an inflammatory cytokine production inhibitor for suppressing inflammatory cytokine production caused by ultraviolet rays, and an inflammation inhibitor for suppressing inflammation caused by ultraviolet rays.
